(19) Europäisches Patentamt / European Patent Office / Office européen des brevets

(11) **EP 2 260 805 B1**

(12) **EUROPÄISCHE PATENTSCHRIFT**

(45) Veröffentlichungstag und Bekanntmachung des Hinweises auf die Patenterteilung:
**22.05.2019 Patentblatt 2019/21**

(51) Int Cl.:
*A61F 9/008* *(2006.01)*   *A61F 9/01* *(2006.01)*
*A61F 9/009* *(2006.01)*

(21) Anmeldenummer: **10182173.4**

(22) Anmeldetag: **23.07.2004**

(54) **Vorrichtung und Verfahren zum Ausbilden gekrümmter Schnittflächen in der Augenhornhaut**

Device and method for forming curved cuts in the cornea

Dispositif et procéde pour former des surfaces de coupe courbes dans la cornée

(84) Benannte Vertragsstaaten:
**AT BE BG CH CY CZ DE DK EE ES FI FR GB GR HU IE IT LI LU MC NL PL PT RO SE SI SK TR**

(30) Priorität: **25.07.2003 DE 10334110**

(43) Veröffentlichungstag der Anmeldung:
**15.12.2010 Patentblatt 2010/50**

(62) Dokumentnummer(n) der früheren Anmeldung(en) nach Art. 76 EPÜ:
**09172932.7 / 2 138 138**
**04763451.4 / 1 648 360**

(73) Patentinhaber: **Carl Zeiss Meditec AG**
**07745 Jena (DE)**

(72) Erfinder:
• **Mühlhoff, Dirk**
  **07751 Jena (DE)**
• **Gerlach, Mario**
  **16548 Glienicke-Nordbahn (DE)**
• **Sticker, Markus**
  **07749 Jena (DE)**
• **Lang, Carsten**
  **07607 Eisenberg (DE)**
• **Bischoff, Mark**
  **07749 Jena (DE)**
• **Bergt, Michael**
  **99425 Weimar (DE)**

(74) Vertreter: **Patentanwälte Geyer, Fehners & Partner mbB**
**Perhamerstrasse 31**
**80687 München (DE)**

(56) Entgegenhaltungen:
**US-A- 5 993 438    US-B1- 6 325 792**

**Beschreibung**

Die Erfindung bezieht sich auf eine Vorrichtung und ein Verfahren zum Ausbilden gekrümmter Schnittflächen in der Augenhornhaut, mit einer Laserstrahlungsquelle, die Laserstrahlung in die Augenhornhaut fokussiert und dort optische Durchbrüche bewirkt, wobei eine Scaneinrichtung, die den Fokuspunkt dreidimensional verstellt, und eine Steuereinrichtung vorgesehen sind, die die Scaneinrichtung ansteuert, um die Schnittfläche durch Aneinanderreihen der optischen Durchbrüche in der Augenhornhaut zu bilden, und wobei die Scaneinrichtung zur Verstellung des Fokuspunktes in einer Raumrichtung eine verstellbare Optik aufweist.

Gekrümmte Schnittflächen innerhalb eines transparenten Materials werden insbesondere bei laserchirurgischen Verfahren und dort insbesondere bei augenchirurgischen Eingriffen erzeugt. Dabei wird Behandlungs-Laserstrahlung innerhalb des Gewebes d.h. unterhalb der Gewebeoberfläche derart fokussiert, dass optische Durchbrüche im Gewebe entstehen.

Im Gewebe laufen dabei zeitlich hintereinander mehrere Prozesse ab, die durch die Laserstrahlung initiiert werden. Überschreitet die Leistungsdichte der Strahlung einen Schwellwert, kommt es zu einem optischen Durchbruch, der im Material eine Plasmablase erzeugt. Diese Plasmablase wächst nach Entstehen des optischen Durchbruches durch sich ausdehnende Gase. Wird der optische Durchbruch nicht aufrechterhalten, so wird das in der Plasmablase erzeugte Gas vom umliegenden Material aufgenommen, und die Blase verschwindet wieder. Dieser Vorgang dauert allerdings sehr viel länger, als die Entstehung der Blase selbst. Wird ein Plasma an einer Materialgrenzfläche erzeugt, die durchaus auch innerhalb einer Materialstruktur liegen kann, so erfolgt ein Materialabtrag von der Grenzfläche. Man spricht dann von Photoablation. Bei einer Plasmablase, die vorher verbundene Materialschichten trennt, ist üblicherweise von Photodisruption die Rede. Der Einfachheit halber werden all solche Prozesse hier unter dem Begriff optischer Durchbruch zusammengefasst, d.h. dieser Begriff schließt nicht nur den eigentlichen optischen Durchbruch sondern auch die daraus resultierenden Wirkungen im Material ein.

Für eine hohe Genauigkeit eines laserchirurgischen Verfahrens ist es unumgänglich, eine hohe Lokalisierung der Wirkung der Laserstrahlen zu gewährleisten und Kollateralschäden in benachbartem Gewebe möglichst zu vermeiden. Es ist deshalb im Stand der Technik üblich, die Laserstrahlung gepulst anzuwenden, so dass der zur Auslösung eines optischen Durchbruchs nötige Schwellwert für die Leistungsdichte nur in den einzelnen Pulsen überschritten wird. Die US 5.984.916 zeigt diesbezüglich deutlich, dass der räumliche Bereich des optischen Durchbruchs (in diesem Fall der erzeugten Wechselwirkung) stark von der Pulsdauer abhängt. Eine hohe Fokussierung des Laserstrahls in Kombination mit sehr kurzen Pulsen erlaubt es damit, den optischen Durchbruch sehr punktgenau in einem Material einzusetzen.

Der Einsatz von gepulster Laserstrahlung hat sich in der letzten Zeit besonders zur laserchirurgischen Fehlsichtigkeitskorrektur in der Ophthalmologie durchgesetzt. Fehlsichtigkeiten des Auges rühren oftmals daher, dass die Brechungseigenschaften von Hornhaut und Linse keine optimale Fokussierung auf der Netzhaut bewirken.

Die erwähnte US 5.984.916 B1 sowie die US 6.110.166 B1 und die US 6.325.792 B1 beschreiben Verfahren zur Schnitterzeugung mittels geeigneter Erzeugung optischer Durchbrüche, so dass im Endeffekt die Brechungseigenschaften der Hornhaut gezielt beeinflusst werden. Eine Vielzahl von optischen Durchbrüchen wird so aneinandergesetzt, dass innerhalb der Hornhaut des Auges ein linsenförmiges Teilvolumen isoliert wird. Das vom übrigen Hornhautgewebe getrennte linsenförmige Teilvolumen wird dann über einen seitlich öffnenden Schnitt aus der Hornhaut herausgenommen. Die Gestalt des Teilvolumens ist so gewählt, dass nach Entnahme die Form und damit die Brechungseigenschaften der Hornhaut so geändert sind, dass die erwünschte Fehlsichtigkeitskorrektur bewirkt ist. Die dabei geforderten Schnittflächen sind gekrümmt, was eine dreidimensionale Verstellung des Fokus nötig macht. Es wird deshalb eine zweidimensionale Ablenkung der Laserstrahlung mit gleichzeitiger Fokusverstellung in einer dritten Raumrichtung kombiniert.

Die US 6.325.792 B1 schlägt weiter die Verwendung eines Kontaktglases vor, das die Hornhautvorderseite stabilisiert und verformt.

Die zweidimensionale Ablenkung der Laserstrahlung ist wie die Fokusverstellung gleichermaßen für die Genauigkeit, mit der die Schnittfläche erzeugt werden kann, ausschlaggebend. Gleichzeitig wirkt sich die Verstellgeschwindigkeit, die dabei erreichbar ist, auf die Schnelligkeit, mit der die geforderte Schnittfläche erzeugt werden kann, aus. Eine schnelle Schnittflächenerzeugung ist nicht nur aus Komfort- oder Zeitersparniswünschen anzustreben, vor dem Hintergrund, dass bei ophthalmologischen Operationen unvermeidlicher Weise Bewegungen des Auges auftreten, fördert eine schnelle Schnittflächenerzeugung zusätzlich die optische Qualität des erzielten Resultats bzw. senkt die Anforderungen an eventuelle Nachführungen von Augenbewegungen.

Der Erfindung liegt deshalb die Aufgabe zugrunde eine Vorrichtung und ein Verfahren der eingangs genannten Art so auszugestalten, dass für die Erzeugung einer Schnittfläche eine möglichst geringe Zeit erforderlich ist.

Die Erfindung ist in den unabhängigen Ansprüchen 1 und 9 definiert.

Bei einer Vorrichtung der eingangs genannten Art steuert die Steuereinrichtung die Scaneinrichtung so, dass der Fokuspunkt in den übrigen zwei Raumrichtungen auf Höhenlinien der Schnittfläche geführt ist, welche in Ebenen liegen, die senkrecht zur ersten Raumrichtung sind.

Erfindungsgemäß werden also bei der Erzeugung der optischen Durchbrüche Bahnen verwendet, denen Höhenlinien der zu erzeugenden Schnittfläche zugrunde liegen. Die Höhenlinien sind dabei auf diejenige Raumrichtung des Systems bezogen, in der die langsamste Verstellgeschwindigkeit gegeben ist. Es ist dadurch möglich, den Fokus in dieser Raumrichtung über einen längeren Zeitraum nahezu unverändert zu lassen, und die höhere Verstellgeschwindigkeit in den anderen beiden Raumrichtungen kann ohne Begrenzung ausgeschöpft werden. Man erhält insgesamt eine schnelle Schnittflächenerzeugung. Die Höhenlinien kann man zweckmäßigerweise durch Schneiden der gekrümmten Schnittfläche mit Ebene senkrecht zur ersten Raumrichtung erhalten. Je exakter die Ebenen der Höhenlinien senkrecht zur ersten Raumrichtung stehen, desto konstanter kann die Verstellung in der ersten Raumrichtung während einer Höhenlinie gehalten werden.

Die Laserstrahlung wird dazu zumindest bezogen auf die zwei Raumrichtungen, die senkrecht zur Ebene der Höhenlinie liegen, unter Berücksichtigung des Höhenlinienverlaufes verstellt. Dabei ist es zum einen möglich, dass der Fokuspunkt innerhalb gewisser Toleranzen exakt der jeweiligen Höhenlinie folgt. In diesem Fall beschreibt der Fokuspunkt konzentrisch gelegene geschlossene Bahnkurven, wobei für jede Bahnkurve der Fokus in der ersten Raumrichtung entsprechend unterschiedlich eingestellt ist. Anstelle von geschlossenen Bahnkurven, die den Höhenlinien innerhalb bestimmter Toleranzen exakt folgen, ist es auch möglich, die Höhenlinien zusammenhängend miteinander zu verbinden. Der Fokuspunkt wird dabei entlang einer Höhenlinie bewegt, wobei die einzelnen Höhenlinien nicht als geschlossene Bahnkurven ausgeführt werden, sondern benachbarte Höhenlinien durch einen gleitenden Übergang miteinander verbunden sind, so dass insgesamt der Fokuspunkt auf einer einzigen zusammenhängenden Bahnkurve bewegt wird. Es entsteht dadurch eine auf einer geschlossenen Bahnkurve liegende Reihe optischer Durchbrüche, die die Schnittfläche bilden. Diese ununterbrochene Aneinanderreihung von Höhenlinien kann vorzugsweise dadurch erreicht werden, dass der Fokuspunkt jeweils bis auf ein Reststück vollständig entlang der Höhenlinie bewegt wird und im Reststück durch Verstellung des Fokuspunktes in der ersten Raumrichtung ein Übergang zur nächsten Höhenlinie bewirkt wird. Dieser Ansatz hat den Vorteil, dass die Anforderungen an die Verstellung in der ersten Raumrichtung nochmals gesenkt sind, da auch während des Übergangs zwischen zwei Höhenlinien optische Durchbrüche zur Bildung der Schnittfläche erzeugt werden.

Das Höhenlinienbild wird dabei von der Topographie, d.h. der Krümmung der Schnittfläche abhängen. Bei einer sphärisch gekrümmten Schnittfläche erhält man konzentrische kreisförmige Höhenlinien. Da bei augenoptischen Korrekturen regelmäßig auch ein gewisser Astigmatismus zu korrigieren ist, wird jedoch eine sphärisch gekrümmte Schnittfläche eher die Ausnahme, eine Ellipsoid- oder Toroidfläche dagegen meist die Regel sein. Für eine solche Ellipsoidfläche sind die Höhenlinien als (günstiger Weise konzentrische) Ellipsen ausgebildet. Vorzugsweise liegt die Elliptizität zwischen 1,0 und 1,1 oder sogar 1,2.

Bei einer solchen Form können die Höhenlinien auch derart der Führung des Fokuspunktes zugrunde gelegt werden, dass der abgelenkte Fokuspunkt einer Ellipsoid-Spirale folgt, d.h. einer auf der Mantelfläche der gekrümmten Schnittfläche liegenden Spirale.

Die Elliptizität der Ellipsen bzw. der Ellipsoid-Spirale kann dabei von der Form der Hornhautoberfläche abhängen. Unter Elliptizität ist das Verhältnis der großen Hauptachse einer Ellipse zu ihrer kleinen Hauptachse zu verstehen.

Aufgrund der erfindungsgemäßen Verwendung eines Kontaktglases spielt die Form dieses Kontaktglases eine Rolle. Hier besteht ein Vorteil des Ansatzes mit Verwendung eines Kontaktglases, da die Topographie mit angepresstem Kontaktglas wohl definiert ist. Ein ebenes Kontaktglas stellt einen mathematischen Grenzfall dar, und das Konzept des Höhenlinien-Scans führt hier zur Entartung der Bahnkurven, obwohl diese auch noch als geschlossen bezeichnet werden können. Für den Fall eines gekrümmten Kontaktglases ergibt sich eine Abhängigkeit der Oberflächentopographie, z. B. der Elliptizität von der Krümmung des Kontaktglases. Dies gilt auch, wenn die Krümmung rein sphärisch ist, da sich dann ebenfalls eine Ellipsoidform für die Schnittfläche einstellt. Die Elliptizität ist allerdings meist nicht im ganzen Bearbeitungsfeld gleich, sondern zeigt oft eine radiale Abhängigkeit.

Im Prinzip ergibt sich für die Elliptizität e folgender Zusammenhang:

$$e(z) = \frac{\sqrt{R_a^2 - (R_a - z)^2}}{\sqrt{R_b^2 - (R_b - z)^2}},$$

wobei $R_a$ und $R_b$ die Krümmungsradien der Cornea-Oberfiäche in Richtung der Hauptachsen der Ellipse bezeichnen und z der Abstand des Bearbeitungspunktes (der Höhenlinie) vom Hornhautscheitel ist. Da z dann eine Funktion des radialen Parameters des Bearbeitungsfeldes (Abstand zur optischen Achse) ist, ist es zweckmäßig, die bereits erwähnte radiale Abhängigkeit der Elliptizität als $e(z) = e(z(r))$ zu wählen.

Die oben genannte Gleichung gilt primär für das unkontaktierte Auge, da auch dort, wie oben erwähnt, meist eine Ellipsoidform vorliegt. Bei Anpressen an ein Kontaktglas kommt es in der Regel zu einer Deformation, die bei der Berechnung berücksichtigt wird. Dabei spielen neben den Kugelkoordinaten $R, \varphi, \alpha$ im natürlichen Augensystem und im Kontaktglassystem (gestrichene Koordinaten) der äußere Krümmungsradius der Cornea $R_{Cv}$ und der Krümmungsradius des Kontaktglases $R_G$ eine Rolle. Die erfindungsgemäße einfache und kompakte Form der Transformationsgleichungen für diese Anpresstransformation lautet:

$$\varphi' = \varphi$$

$$\alpha' \cdot R' = \alpha \cdot R$$

$$R_G - R' = R_{Cv} - R$$

Weitere Ergänzungen, die in den Gleichungen zu Korrekturtermen führen, sind natürlich möglich und teilweise auch sinnvoll. Der hier offenbarte heuristische Ansatz wird dadurch jedoch nur modifiziert, gilt also im Prinzip weiter. Die genannten Zusammenhänge ermöglichen eine einfache Berechnung der Bahnkurven, was auch die Berechnung der Elliptizität mit einschließt. Ein besonders wichtiger Schritt bei den Algorithmen zur Berechnung ist die oben angegebene Vorwärts- und Rückwärtstransformation zwischen natürlichem Augensystem und Kontaktglassystem.

Die Elliptizität der Bahnkurven beträgt für ein Kontaktglas mit einem Krümmungsradius, der etwa dem des menschlichen Auges entspricht, in der Regel weniger als 1.4 (große Hauptachse 10% länger als die kleine Hauptachse). Die Elliptizität beträgt bei einer sphäro-zylindrischen Korrektur mit -2dpt und 1dpt beispielsweise nur etwa 1.03 im zentralen Feldbereich nahe der optischen Achse und wächst mit dem Abstand von der optischen Achse bis zur äußeren Bahnkurve um ca. 10%. Für eine praktikable Ausführungsform spielt die Veränderlichkeit der Elliptizität oder einer entsprechenden Modifikation einer idealen Kreisbahn bei Fehlsichtigkeitskorrektur höherer Ordnung keine störende Rolle, kann also in einer ersten Näherung als konstant angenommen werden.

Die Abstände zwischen den der Steuerung zugrunde zu legenden Höhenlinien sind naturgemäß durch die Abstände der Ebenen gegeben, die durch einen mathematischen Schnitt mit der gekrümmten Schnittfläche die Höhenlinien erzeugt sind. Um sicherzustellen, dass die Vielzahl von optischen Durchbrüchen eine zusammenhängende Schnittfläche ausbildet, ist darauf zu achten, dass der Maximalabstand der Höhenlinien einen Grenzwert nicht überschreitet. Es ist deshalb zweckmäßigerweise zu bevorzugen, dass Abstände der Höhenlinien in der ersten Raumrichtung so gewählt werden, dass die Abstände zwischen benachbarten Höhenlinien einen Grenzwert nicht überschreiten. Dabei kann als zu überprüfendes Maß sowohl der Abstand im Höhenlinienbild als auch der Abstand im dreidimensionalen Raum verwendet werden. Da in der Augenchirurgie die gekrümmten Schnittflächen zur optischen Korrektur innerhalb gewisser Grenzen in oftmals ausreichender Näherung einer sphärischen bzw. einer Ellipsoid-Geometrie genügen, kann es zur Vereinfachung ausreichen, dass die Abstände in der ersten Raumrichtung so gewählt werden, dass die mittleren Abstände der Höhenlinien konstant sind und insbesondere unterhalb eines Schwellwertes liegen, der natürlich geringer ist als der vorgenannte Grenzwert. Bei ellipsoidförmigen Schnittflächen kann vereinfacht im Höhenlinienbild der Abstand benachbarter Höhenlinien an der langen Halbachse ausgewertet werden, um sicherzustellen, dass die optischen Durchbrüche ausreichend dicht liegen.

Bei ophthalmologischen Operationen kann es mitunter erforderlich werden, auch höhere Aberrationen durch Entfernen eines Volumens aus der Hornhaut zu korrigieren. Die dazu erforderliche gekrümmte Schnittfläche weist dementsprechend dann auch höhere Krümmungsordnungen auf. Will man diese Formen durch Höhenlinien direkt abbilden, ergibt sich mitunter ein sehr komplexes Höhenlinienbild, das eine komplexe und schnelle Verstellung in den zwei übrigen Raumrichtungen beim Abfahren einer Höhenlinie erfordert. Für solche Fälle ist es zweckmäßig, bei der Bestimmung der Höhenlinien die höheren Krümmungsordnungen der gekrümmten Schnittfläche zu vernachlässigen und dann, während der Fokuspunkt in den übrigen zwei Raumrichtungen gemäß der Höhenlinie verstellt wird, die Verstellung in der ersten Raumrichtung gemäß dem Einfluss der höheren Krümmungsordnungen zu verändern. Die Korrektur höherer Aberrationen wird also dann in der ersten Raumrichtung, z.B. in z-Richtung auf eine Grundbewegung aufmoduliert, die der gekrümmten Schnittfläche ohne höhere Aberrationen entspricht.

Bei vielen augenoptischen Korrekturen ist es aufgrund physiologischer Gegebenheiten vorteilhaft, zur Fehlsichtigkeitskorrektur ein Volumen zu entnehmen, das bezogen auf die optische Achse des Auges in einem kreisförmig begrenzten Bereich liegt. Dies gilt auch, falls astigmatische Korrekturen nötig sind. Für solche Fälle ist es vorteilhaft, mittels der Höhenlinien eine Ellipse abzutasten, in den Randbereichen, in denen die Ellipse über den gewünschten kreisförmigen Bereich hinausragt, jedoch die Laserstrahlung (z. B. durch einen optischen Schalter oder eine Blende oder durch Eingriff an der Laserstrahlungsquelle) so zu steuern, dass dort keine optischen Durchbrüche bewirkt werden. Durch eine derartige Ausblendung von Randbereichen der Ellipse kann sichergestellt werden, dass die (astigmatisch) gekrümmte Schnittfläche nur in einem kreisförmigen Bereich erzeugt wird.

In der erfindungsgemäßen Vorrichtung kann die Verstellung des Fokuspunktes mit einer Scaneinrichtung bewirkt werden, die zur Verstellung in der ersten Raumrichtung (üblicherweise z-Richtung) ein vorzugsweise als abstimmbares Teleskop ausgebildetes Zoom-Objektiv und für die anderen beiden Raumrichtungen (üblicherweise x- und y-Richtungen) zwei Kippspiegel mit gekreuzten Drehachsen aufweist.

Für die durch optische Mittel bewirkte Ausbildung gekrümmter Schnittflächen ist es vorteilhaft, wenn die Oberfläche des Materials, insbesondere die Augenhornhautvorderfläche, eine bekannte Form hat. Dies erleichtert die Führung des Fokuspunktes. Weiter ist es zweckmäßig, das zu bearbeitende Material, insbesondere die Augenhornhaut, räumlich zu fixieren, da man dann auf mitunter aufwendige Strahlnachführungen verzichten kann. Unter beiden Gesichtspunkten ist es zweckmäßig, auf das Material ein Kontaktglas aufzusetzen, das der Materialoberfläche eine bestimmte Form gibt. Diese Form wird dann bei der Bestimmung der Höhenlinien berücksichtigt. Dies kann insbesondere dadurch erfolgen, dass die eingangs erwähnte Koordinatentransformation, die durch das Anpressen an das Kontaktglas erfolgt, bei der Ansteuerung eingeht.

Die Verwendung eines Kontaktglases ist für die erfindungsgemäße Vorrichtung vorteilhaft. Bei der Vorrichtung ist die vom Kontaktglas der Oberfläche des Materials verliehene Form in der Steuereinrichtung bekannt oder wird dieser geeignet eingegeben, so dass die Steuereinrichtung bei der Wahl der Höhenlinien die Oberflächenform des Materials zugrunde legt.

Die Erfindung wird nachfolgend unter Bezugnahme auf die Zeichnung beispielhalber noch näher erläutert. In der Zeichnung zeigt:

| Figur 1 | eine perspektivische Darstellung eines Patienten während einer laserchirurgischen Behandlung mit einem laserchirurgischen Instrument, |
| Figur 2 | die Fokussierung eines Strahlenbündels auf das Auge des Patienten beim Instrument der Figur 1, |
| Figur 3 | eine schematische Darstellung zur Erläuterung einer während der laserchirurgischen Behandlung mit dem Instrument der Figur 1 erzeugten Schnittfläche, |
| Figur 4 | eine Ablenkvorrichtung des laserchirurgischen Instruments der Figur 1, |
| Figur 5 | ein beispielhaftes Höhenlinienbild, das bei der Ansteuerung der Ablenkeinrichtung der Figur 4 zugrunde gelegt wird, |
| Figur 6 | einen Ausschnitt eines Höhenlinienbilds ähnlich dem der Figur 5 zur Verdeutlichung des Übergangs zwischen aufeinanderfolgenden Höhenlinien, |
| Figur 7 | ähnlich der Figur 6 mit einer weiteren Möglichkeit für einen Übergang zwischen Höhenlinien, |
| Figuren 8a und 8b | ein weiteres Beispiel für ein Höhenlinienbild samt zugehörigen Ansteuerfunktionen für die Ablenkvorrichtung der Figur 4, |
| Figur 9 | eine Draufsicht auf einen Schnittbereich beim Ausführen einer augenoptischen Operation zur Fehlsichtigkeitskorrektur, |
| Figur 10 | eine Darstellung ähnlich der Figur 2 bei Verwendung eines Kontaktglases, |
| Figur 11 | bei der Bestimmung der Höhenlinien relevante Größen und |
| Figuren 12 und 13 | die Größen der Figur 11 mit und ohne Kontaktglas. |

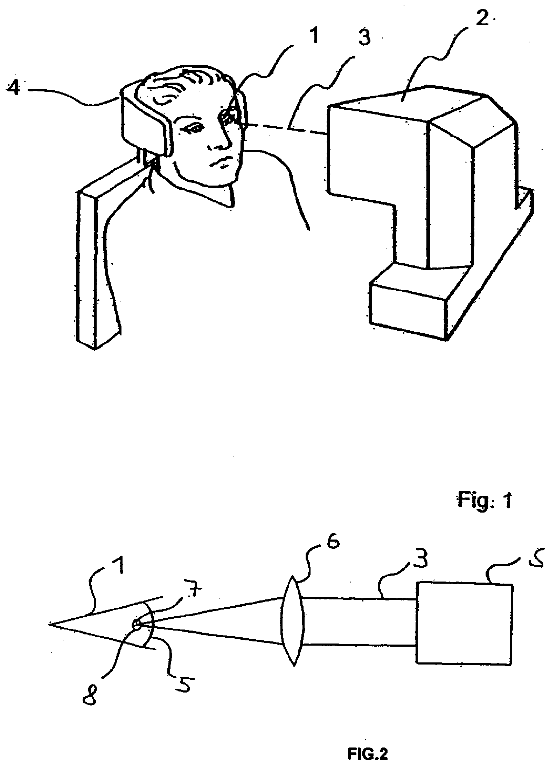

In Figur 1 ist ein laserchirurgisches Instrument zur Behandlung eines Auges 1 eines Patienten gezeigt, wobei das laserchirurgische Instrument 2 zur Ausführung einer refraktiven Korrektur dient. Das Instrument 2 gibt dazu einen Behandlungs-Laserstrahl 3 auf das Auge des Patienten 1 ab, dessen Kopf in einen Kopfhalter 4 fixiert ist. Das laserchirurgische Instrument 2 ist in der Lage, einen gepulsten Laserstrahl 3 zu erzeugen, so dass das in US 6.110.166 beschriebene Verfahren ausgeführt werden kann.

Das laserchirurgische Instrument 2 weist dazu, wie in Figur 2 schematisch dargestellt ist, eine Strahlquelle S auf, deren Strahlung in die Hornhaut 5 des Auges 1 fokussiert wird. Mittels des laserchirurgischen Instrumentes 2 wird eine Fehlsichtigkeit des Auges 1 des Patienten dadurch behoben, dass aus der Hornhaut 5 Material so entfernt wird, dass sich die Brechungseigenschaften der Hornhaut um ein gewünschtes Maß ändern. Das Material wird dabei dem Stroma der Hornhaut entnommen, das unterhalb von Epithel und Bowmanscher Membran oberhalb der Decemetschen Membran und des Endothels liegt.

Die Materialentfernung erfolgt, indem durch Fokussierung des hochenergetischen gepulsten Laserstrahls 3 mittels eines objektiven Teleskops 6 in einem in der Hornhaut 5 liegenden Fokus 7 in der Hornhaut Gewebeschichten getrennt werden. Jeder Puls der gepulsten Laserstrahlung 3 erzeugt dabei einen optischen Durchbruch im Gewebe, welcher eine Plasmablase 8 initiiert. Dadurch umfasst die Gewebeschichttrennung ein größeres Gebiet, als der Fokus 7 der Laserstrahlung 3. Durch geeignete Ablenkung des Laserstrahls 3 werden nun während der Behandlung viele Plasmablasen 8 aneinandergereiht. Die aneinander liegenden Plasmablasen 8 bilden dann eine Schnittfläche 9, die ein Teilvolumen T des Stromas, nämlich das zu entfernende Material der Hornhaut 5 umschreiben.

Das laserchirurgische Instrument 2 wirkt durch die Laserstrahlung 3 wie ein chirurgisches Messer, das, ohne die Oberfläche der Hornhaut 5 zu verletzen, direkt Materialschichten im Inneren der Hornhaut 5 trennt. Führt man den Schnitt durch weitere Erzeugung von Plasmablasen 8 bis an die Oberfläche der Hornhaut 5, kann ein durch die Schnittfläche 9 isoliertes Material der Hornhaut 5 seitlich herausgezogen und somit entfernt werden.

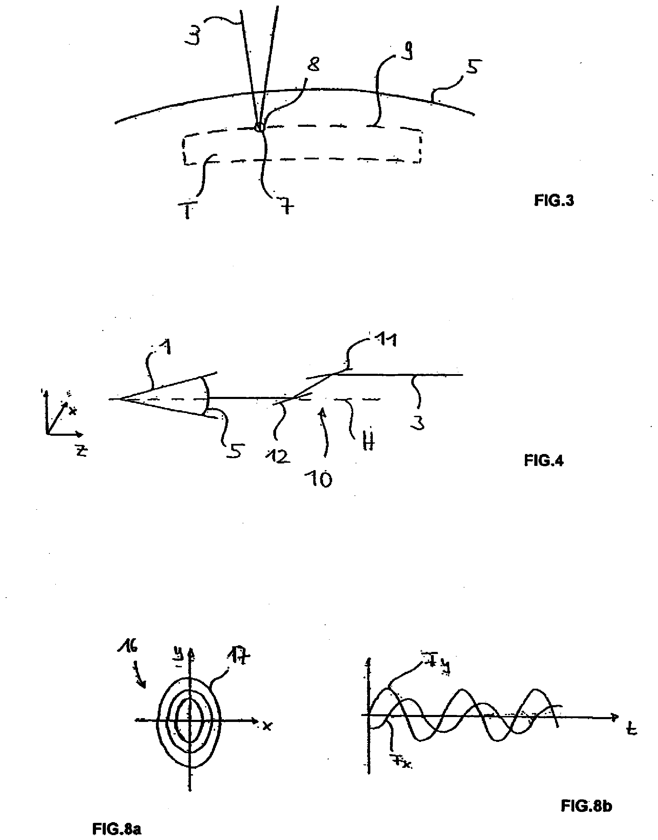

Die Erzeugung der Schnittfläche 9 mittels des laserchirurgischen Instrumentes 2 ist in Figur 3 schematisch dargestellt. Durch Aneinanderreihung der Plasmablasen 8 in Folge stetiger Verschiebung des Fokus 7 des gepulsten fokussierten Laserstrahls 3 wird die Schnittfläche 9 gebildet.

Die Fokusverschiebung erfolgt dabei zum einen in einer Ausführungsform mittels der in Figur 4 schematisch dargestellten Ablenkeinheit 10, die den auf einer Haupteinfallsachse H auf das Auge 1 einfallenden Laserstrahl 3 um zwei senkrecht zueinander liegenden Achsen ablenkt. Die Ablenkeinheit 10 verwendet dafür einen Zeilenspiegel 11 sowie einen Bildspiegel 12, was zu zwei hintereinander liegenden räumlichen Ablenkachsen führt. Der Kreuzungspunkt der Hauptstrahlachse mit der Ablenkachse ist dann der jeweilige Ablenkpunkt. Zur Fokusverschiebung wird zum anderen das Teleskop 6 geeignet verstellt. Dadurch kann der Fokus 7 in dem in Figur 4 schematisch dargestelltem x/y/z-Koordinatensystem entlang dreier orthogonaler Achsen verstellt werden. Die Ablenkeinheit 10 verstellt den Fokus in der x/y-Ebene, wobei der Zeilenspiegel den Fokus in der x-Richtung und der Bildspiegel in der y-Richtung zu verstellen erlaubt. Das Teleskop 6 wirkt dagegen auf die z-Koordinate des Fokus 7.

Ist eine wie in Fig. 3 gezeigte Schnittfläche 9 in die gleiche Richtung wie die Hornhautoberfläche gewölbt, so ist dies mit einer Optik, deren Bildfeldkrümmung ähnlich der Krümmung der Hornhaut ist, zu erreichen, ohne dass die Führung des Fokus 7 dies berücksichtigen muss.

Aufgrund der Corneakrümmung, die zwischen 7 und 10 mm beträgt, ist das Teilvolumen T auch entsprechend gekrümmt. Die Corneakrümmung wirkt sich somit in Form einer Bildfeldkrümmung aus. Diese wird durch geeignete Ansteuerung der Ablenkeinheit berücksichtigt.

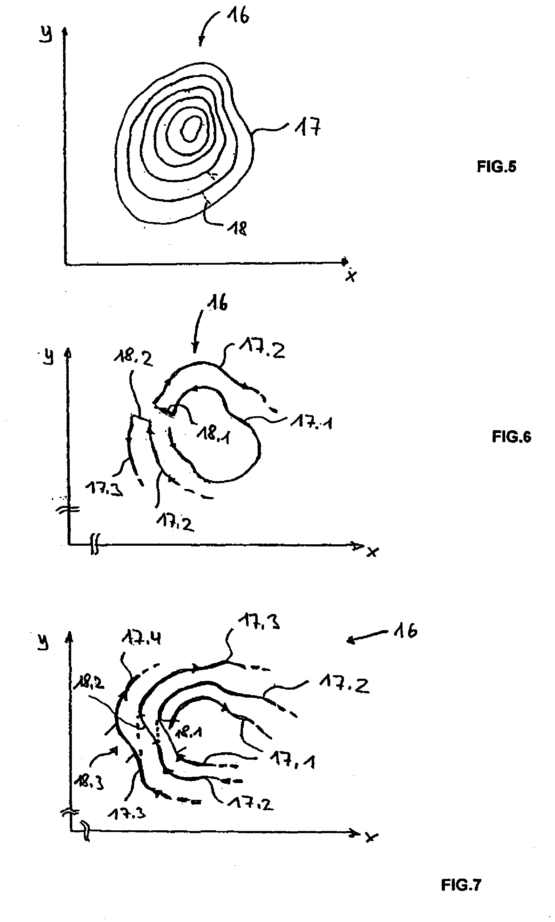

Zur Erzeugung der Schnittfläche 9 wird aus deren Krümmung ein Höhenlinienbild 16 bestimmt, wie es beispielshalber in Figur 5 in der x/y-Ebene dargestellt ist. Das Höhenlinienbild 16 besteht aus einer Vielzahl konzentrischer Höhenlinien 17, die Punkte gleicher z-Koordinaten der Schnittfläche 9 verbindet. Das Höhenlinienbild 16 wurde gewonnen, in dem aus der gekrümmten Schnittfläche 9 diejenigen Punkte bestimmt, z. B. herausgefiltert wurden, die zumindest näherungsweise eine bestimmte z-Koordinate haben. Dies entspricht einem mathematischen Schnitt der gekrümmten Schnittfläche 9 mit einer x/y-Ebene mit der jeweiligen z-Koordinate. Die z-Koordinaten wurden dabei zur Erzeugung der einzelnen Höhenlinien 17 des Höhenlinienbildes 16 der Figur 5 so gewählt, dass die Abstände benachbarter der Höhenlinien 17 im Höhenlinienbild 16 einen vorbestimmten Grenzwert nicht überschreiten. Dieser Grenzwert ist durch den maximal zulässigen Abstand zweier Plasmablasen 8 festgelegt, der zum Erreichen einer zusammenhängenden Schnittfläche zulässig ist.

Zum Erzeugen der Schnittfläche 9 wird nun der Fokus 7 entsprechend der Höhenlinien 17 durch die Ablenkeinheit 10 verstellt, wobei die Zoom-Optik 6 für jede Höhenlinie 17 die entsprechende z-Koordinate für den Fokus 7 einstellt. Während der Fokus 7 über eine Höhenlinie 17 läuft, bleibt das Teleskop 6 fest eingestellt. Lediglich während in Figur 5 gestrichelt eingezeichneten Übergängen 18 zwischen benachbarten Höhenlinien erfolgt eine Verstellung.

Figur 6 zeigt einen Ausschnitt des Höhenlinienbildes 16. Jede Höhenlinie 17 wird dabei als fast vollständig geschlossene Kurve vom Fokus 7 abgefahren, wobei der Abstand zwischen Anfang und Ende einer Höhenlinie 17 den durch den Grenzwert definierten zulässigen maximalen Abstand zwischen zwei Plasmablasen 8 nicht überschreitet. Am Ende einer jeden Höhenlinie 17 (in Figur 6 sind drei Höhenlinien 17.1, 17.2 und 17.3 angedeutet) erfolgt ein Übergang 18 durch Verstellen des Teleskopes 6 zur jeweils nächsten Höhenlinie. Zwischen den Höhenlinien 17.1 und 17.2 liegt dadurch ein Übergang 18.1, zwischen den Höhenlinien 17.2 und 17.3 ein Übergang 18.2. Dies setzt sich für alle Höhenlinien fort. Durch den derart gewählten Übergang ist zum einen erreicht, dass der Grenzwert für den maximal zulässigen Abstand zwischen zwei Plasmablasen 8 nicht überschritten wird, zum anderen die Höhenlinien 17 als zusammenhängende Spur geschrieben werden kann.

In Figur 6 liegen die Übergänge 18 im Wesentlichen auf Falllinien der gekrümmten Schnittfläche 9. Figur 7 zeigt diesbezüglich andere Übergänge 18.1 bis 18.3, bei denen ein gleitender Übergang zwischen dem Ende einer Höhenlinie und dem Beginn der unmittelbar benachbarten Höhenlinie erfolgt. Zur Verdeutlichung ist die vom Fokus 7 nicht verfolgte Fortsetzung der entsprechenden Höhenlinien in Figur 7 gestrichelt eingezeichnet. Wie zu sehen ist, wird am Ende einer Höhenlinie 17 ein gleitender Übergang auf die nächste Höhenlinie durch geeignete Ansteuerung des Zeilenspiegels 11 sowie des Bildspiegels 12 vorgenommen. Gleichzeitig wird synchron das Teleskop 6 während der dadurch erreichten Übergänge 18.1, 18.2 und 18.3 verstellt.

Es ergibt sich dadurch im Gegensatz zum Übergang der Figur 6, bei der benachbarte Höhenlinien in entgegengesetzter Umlaufrichtung durchlaufen werden, ein gleichsinniger Umlauf um die Höhenlinien, die ähnlich einer Spirale aneinandergereiht werden. Im Unterschied zu einer wirklichen Spirale wird jedoch bis auf den Übergang 18 die Höhenlinie durch den Fokus 7 abgefahren und der Wechsel von einer Höhenlinie zur nächsten erfolgt über einen kleinen Winkelbereich des Umlaufs statt kontinuierlich während eines 360° Umlaufes.

Figur 8a zeigt ein weiteres Beispiel für ein Höhenlinienbild 16, das hier aus konzentrischen elliptischen Höhenlinien 17 aufgebaut ist. Für dieses Höhenlinienbild ist für jede Höhenlinie 17 die in Figur 8b schematisch dargestellte zeitliche Ansteuerung von Zeilenspiegel 11 und Bildspiegel 12 vorgesehen, die hier mit Ansteuerfunktionen Fy und Fx angesteuert werden, die der Gleichung sin φ bzw. A • sin(φ+α) und cos φ bzw. R • cos(φ+α) genügen (mit φ Winkelparameter der Höhenlinie, α auf die Winkellage der Ellipsenhauptachse zur y-Achse wirkendem Parameter R, und A die Elliptizität beeinflussendem Parameter, wobei meist R=1 gilt).

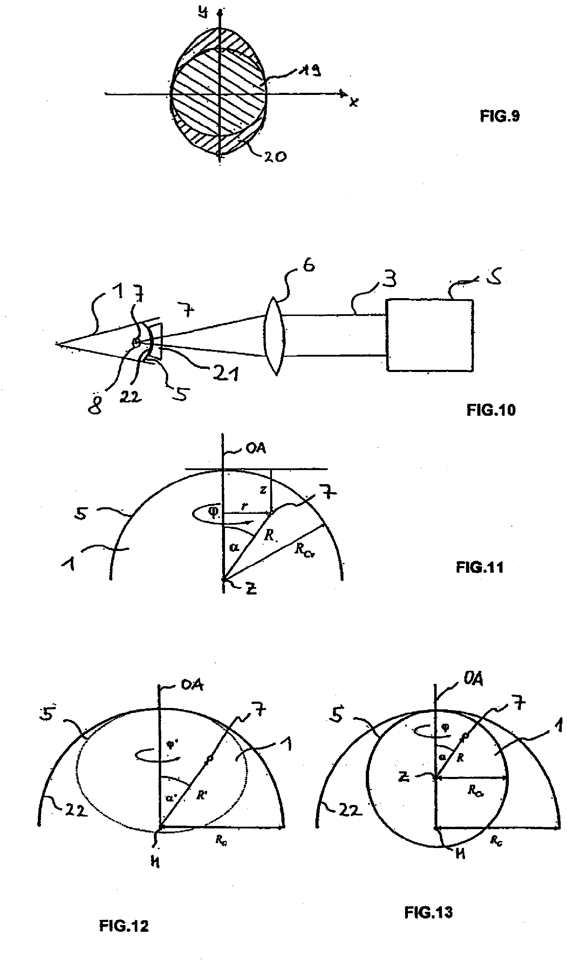

Da bei einem nichtkreisförmigen Höhenlinienbild die Schnittfläche 9, in z-Richtung gesehen einen nichtkreisförmigen Bereich umfasste, was aus ophthalmologischer Sicht nicht wünschenswert ist, wird in einer Ausführungsform bei solchen, nicht rotationssymmetrischen Höhenlinienbildern in Bereichen, die außerhalb eines kreisförmigen Bereiches liegen, die Strahlquelle S so gesteuert, dass sie im Material 5 keinen optischen Durchbruch, d.h. keine Plasmablase 8 erzeugt. Dies ist in Figur 9 durch unterschiedliche Schraffuren dargestellt. Im von links oben nach rechts unten schraffierten kreisförmigen Bereich 19 kann die Strahlquelle S Plasmablasen 8 erzeugen. In den darüber hinausragenden Bereichen 20, in denen das Höhenlinienbild 16 den gewünschten kreisförmigen Bereich 19 verlässt, ist die Strahlquelle S dagegen abgeschaltet oder wird zumindest so betrieben, dass keine Plasmablasen 8 entstehen können.

Bisher wurde das laserchirurgische Instrument 2 sowie das dadurch ausgeführte Verfahren in Zusammenhang mit einem Konzept beschrieben, das die Form der Vorderfläche der Augenhornhaut für den Eingriff unverändert lässt. Obige Beschreibung gilt jedoch auch, obwohl die Ausführungsformen der Erfindung auf die Augenhornhaut 5 ein Kontaktglas aufsetzen. Der dabei vorliegende Aufbau ist schematisch in Figur 10 gezeigt, die im Wesentlichen der Figur 2 entspricht, so dass auf dort bereits beschriebene Elemente nicht weiter eingegangen wird. Auf die Augenhornhaut 5 ist ein Kontaktglas 21 aufgesetzt, das mit seiner Innenfläche 22 der Vorderfläche der Augenhornhaut 5 ein bestimmtes Profil verleiht. Bei der Bestimmung der Bahnkurven, z. B. der Höhenlinien, ist deshalb nicht die Krümmung der Augenhornhaut 5 im freien, d. h. natürlichen Zustand, zugrunde zu legen, sondern die durch die Innenfläche 22 des Kontaktglases 21 vorgegebene Form.

Ohne Kontaktglas 21 stellen sich die geometrischen Verhältnisse am Auge 1 wie in Figur 11 gezeigt dar. Die Augenhornhaut 5 ist bezogen auf das Augenzentrum Z näherungsweise sphärisch gekrümmt, so dass sie durch einen Krümmungsradius $R_{Cv}$ sowie die Lage des Zentrums Z auf der optischen Achse OA eindeutig lagebestimmt ist. Die Koordinaten eines Punktes, an dem ein Laserfokus 7 zu liegen kommt, um eine Plasmablase 8 zu erzeugen, sind damit entweder in Zylinderkoordinaten (Radius r von der optischen Achse OA, Abstand z von der Scheitelpunktebene und Winkel φ) oder in radialen Koordinaten (Radius r vom Augenzentrum Z, Winkel φ und α) eindeutig angebbar. In beiden Koordinatensystemen können die Höhenlinien bzw. die Bahnkurven, an denen der Fokus 7 verstellt wird, berechnet und angegeben werden, wobei in Zylinderkoordinaten elliptische Bahnkurven besonders einfach mathematisch beschrieben werden können.

Setzt man nun auf das Auge ein Kontaktglas 21 auf, liegen die in Figur 13 gezeigten Verhältnisse vor, solange das Kontaktglas 21 mit seiner Innenfläche 22 die Augenhornhaut nicht verformt. Das Kontaktglas ist hier sphärisch gekrümmt, wobei der Krümmungsradius $R_G$ größer ist, als der Krümmungsradius $R_{Cv}$ der Augenhornhaut. Presst man nun das Kontaktglas 21 auf das Auge 1, verformt sich die Augenhornhaut 5 von einer Sphäre zu einem Ellipsoid; es stellen sich die in Figur 12 schematisch dargestellten Verhältnisse ein. Das Anpressen bewirkt also die Verformung des Auges, das sich zumindest in einem Bereich um die optische Achse OA sehr viel enger an die Innenfläche 22 des Kontaktglases 21 anlegt, als ohne Anpressung.

Da sich die geometrischen Verhältnisse nun ändern, kann man den Vorgang des Anpressens bezüglich der mathematischen Beschreibung der Orte der Fokuspunkte 7 und damit der Bahnkurven in einer Koordinatentransformation, die auch als "Anpresstransformation" bezeichnet wird, fassen. Die transformierten Koordinaten werden dann zweckmäßigerweise auf den Mittelpunkt M des vorzugsweise sphärisch gekrümmten Kontaktglases bezogen, da das Kontaktglas üblicherweise auch zur Fixierung des Auges 1 verwendet wird, d. h. fest mit dem Instrument 2 verbunden ist. Hier wirkt sich die Doppelfunktion des Kontaktglases (Formgebung und räumliche Fixierung) aus.

Es stellen sich dann elliptische Bahnkurven ein. Die Elliptizität der Bahnkurven hängt von der Form diese Kontaktglases ab. Unter Elliptizität ist das Verhältnis der großen Hauptachse einer Ellipse zu ihrer kleinen Hauptachse zu verstehen.

Ein ebenes Kontaktglas stellt einen mathematischen Grenzfall dar, und das Konzept des Höhenlinien-Scans führt hier zur Entartung der Bahnkurven, obwohl diese auch noch als geschlossen bezeichnet werden können. Für den auch aus applikativer Sicht interessanteren Fall eines gekrümmten Kontaktglases ergibt sich eine Abhängigkeit der Elliptizität von der Krümmung des Kontaktglases. Die Elliptizität ist zudem meist nicht im ganzen Bearbeitungsfeld gleich, sondern zeigt eine radiale Abhängigkeit.

Im Prinzip gilt für die Elliptizität e:

$$e(z) = \frac{\sqrt{R_a^2 - (R_a - z)^2}}{\sqrt{R_b^2 - (R_b - z)^2}},$$

wobei $R_a$ und $R_b$ die Krümmungsradien der Cornea-Oberfläche in Richtung der Hauptachsen der Ellipse bezeichnen und z den Abstand des Bearbeitungspunktes (der Höhenlinie) vom Hornhautscheitel. Da z in dem gewählten Zylinderkoordinatensystem (z, Abstand von Hornhautscheitel; r, Abstand zur optischen Achse; $\varphi$) dann eine Funktion des radialen Parameters v des Bearbeitungsfeldes ist, ist es zweckmäßig, die radiale Abhängigkeit der Elliptizität durch $e(z) = e(z(r))$ zu beschreiben.

Die oben genannte Gleichung gilt primär für das unkontaktierte Auge. Bei Anpressen an ein Kontaktglas kommt es in der Regel zu einer Deformation, die bei der Berechnung berücksichtigt werden muss. Dabei spielen der äußere Krümmungsradius der Cornea $R_{Cv}$ und der Krümmungsradius des Kontaktglases $R_G$ eine Rolle. Eine einfache und kompakte Form der Transformation lautet:

$$\varphi' = \varphi$$

$$\alpha' \cdot R' = \alpha \cdot R$$

$$R_G - R' = R_{Cv} - R$$

Weitere Ergänzungen, die in den Gleichungen zu Korrekturtermen führen, sind natürlich möglich und teilweise auch sinnvoll. Obiger Ansatz wird dadurch jedoch nur modifiziert, gilt also im Prinzip weiter. Die genannten Zusammenhänge ermöglichen die Berechnung der Bahnkurven, was auch die Berechnung der Elliptizität mit einschließt. Ein besonders wichtiger Schritt bei den Algorithmen zur Berechnung ist die Vorwärts- und Rückwärtstransformation zwischen natürlichem Augensystem und Kontaktglassystem.

Die Elliptizität der Bahnkurven beträgt für ein Kontaktglas mit einem Krümmungsradius, der etwa dem des menschlichen Auges entspricht, in der Regel weniger als 1.2 (große Hauptachse 10% länger als die kleine Hauptachse). Die Elliptizität beträgt bei einer sphäro-zylindrischen Korrektur mit -2dpt und 1dpt beispielsweise nur etwa 1.03 im zentralen Feldbereich nahe der optischen Achse und wächst mit dem Abstand von der optischen Achse bis zur äußeren Bahnkurve um ca. 10%. Für einen Ansatz spielt die Veränderlichkeit der Elliptizität oder einer entsprechenden Modifikation einer idealen Kreisbahn bei Fehlsichtigkeitskorrektur höherer Ordnung keine störende Rolle, kann also in einer ersten Näherung als konstant angenommen werden.

Mit (ebenem oder gekrümmtem) Kontaktglas entfällt jeglicher Nachführungsbedarf und es besteht keine Unsicherheit hinsichtlich der vorliegenden Oberflächentopographie.

Ist die Form der Kontaktglasfläche nicht durch eine Sphäre beschreibbar, sondern folgt einer anderen Raumflächenfunktion, beispielsweise einem Paraboloiden, kann analog der oben angegebenen Transformation ein Transformationsgesetz angegeben werden, das aber den gleichen physikalischen Gedanken folgt.

**Patentansprüche**

1. Vorrichtung zum Ausbilden einer gekrümmten Schnittfläche (9) in der Augenhornhaut (5), mit

- einer Laserstrahlungsquelle (S), die Laserstrahlung (3) in die Augenhornhaut (5) fokussiert und dort optische Durchbrüche (8) bewirkt, wobei ein gekrümmtes Kontaktglas (21) zum Anpressen auf eine Oberfläche der Augenhornhaut (5) diese in eine bestimmte gekrümmte Form verformt,
- eine Scaneinrichtung (6, 10), die den Fokuspunkt (7) dreidimensional verstellt, und
- eine Steuereinrichtung (2) vorgesehen sind, die die Scaneinrichtung (6, 10) ansteuert, um die Schnittfläche (9) durch Aneinanderreihen der optischen Durchbrüche (8) in der Augenhornhaut (5) zu bilden,
- wobei die Scaneinrichtung (6, 10) zur Verstellung des Fokuspunktes (7) in einer ersten Raumrichtung (z) eine verstellbare Optik (6) aufweist, und wobei die Steuereinrichtung (2) die Scaneinrichtung (6, 10) so ansteuert, dass der Fokuspunkt auf Basis von Höhenlinien geführt wird, welche in Ebenen liegen, die senkrecht zur ersten Raumrichtung (z) stehen,
- wobei die Steuereinrichtung (2) die Scaneinrichtung (6, 10) so ansteuert, dass die Steuereinrichtung (2) die durch das Kontaktglas (21) bewirkte Verformung der Augenhornhaut (5) in die gekrümmte Form durch eine Koordinatentransformation berücksichtigt, die lautet

$$\varphi' = \varphi$$

$$\alpha' \cdot R' = \alpha \cdot R$$

$$R_G - R' = R_{Cv} - R \ ,$$

wobei $\alpha$, $\varphi$, R Kugelkoordinaten vor der Verformung der Augenhornhaut (5), $\alpha'$, $\varphi'$, R' Kugelkoordinaten nach der Verformung der Augenhornhaut (5), $R_{Cv}$ ein Krümmungsradius der Oberfläche der unverformten Augenhornhaut (5) und $R_G$ ein Krümmungsradius des Kontaktglases (21) sind.

2. Vorrichtung nach Anspruch 1, **dadurch gekennzeichnet, dass** die Steuereinrichtung (2) die Scaneinrichtung (6, 10) so ansteuert, dass der Fokuspunkt in den übrigen zwei Raumrichtungen auf konzentrischen Höhenlinien der Schnittfläche geführt ist.

3. Vorrichtung nach Anspruch 1, **dadurch gekennzeichnet, dass** die Steuereinrichtung (2) die Scaneinrichtung (6, 10) so ansteuert, dass die einzelnen Höhenlinien nicht als geschlossene Kurven ausgeführt werden und benachbarte Höhenlinien durch einen gleitenden Übergang (18.1, 18.2, 18.3) verbunden sind.

4. Vorrichtung nach Anspruch 2 oder 3, **dadurch gekennzeichnet, dass** die Steuereinrichtung (2) die Abstände der Höhenlinien (17) in der ersten Raumrichtung so wählt, dass die mittleren Abstände zwischen benachbarten der Höhenlinien (17) im Wesentlichen, insbesondere innerhalb $\pm$ 10%, konstant sind.

5. Vorrichtung nach Anspruch 1, **dadurch gekennzeichnet, dass** die Steuereinrichtung (2) die Scaneinrichtung (6, 10) so ansteuert, dass der Fokuspunkt (7) einer Ellipsoid-Spirale auf Basis der Höhenlinien folgt.

6. Vorrichtung nach Anspruch 3 bis 5, dadurch gegenzeichnet, dass die Steuereinrichtung (2) bei höheren Krümmungsordnungen der Schnittfläche (9) die Höhenlinien (17) durch Schneiden einer um höhere Krümmungsordnungen bereinigten gekrümmten Schnittflächen (9) mit Ebenen senkrecht zur ersten Raumrichtung (z) bestimmt.

7. Vorrichtung nach Anspruch 6, dadurch gegenzeichnet, dass die Steuereinrichtung (2) die Verstellung in der ersten Raumrichtung (z) gemäß dem Einfluss der höheren Krümmungsordnungen verändert, während sie den Fokuspunkt (7) in den übrigen zwei Raumrichtungen (x, y) gemäß der Höhenlinien (17) verstellt, die der bereinigten Schnittfläche (9) ohne höhere Krümmungsordnungen zugeordnet sind.

8. Vorrichtung nach einem der obigen Ansprüche, **dadurch gekennzeichnet, dass** die Scaneinrichtung (6, 10) zwei Kippspiegel (11, 12) mit gekreuzten Drehachsen aufweist, um die Verstellung in den übrigen zwei Raumrichtungen (x, y) zu bewirken.

9. Verfahren zum Erzeugen von Steuerdaten für eine augenchirurgische Lasereinrichtung (2) vor einem chirurgischen Eingriff, wobei die Steuerdaten Zielkoordinaten vorgeben, die durch eine Koordinatentransformation eine Verformung der Augenhornhaut (5) durch ein gekrümmtes Kontaktglas (21) berücksichtigen, und wobei eine Bahnkurve (17, 17.1, 17.2, 17.3) berechnet wird, die zum Ausbilden einer gekrümmten Schnittfläche (9) in der Augenhornhaut (5) dient, wobei zum Ausbilden der Schnittfläche (9) optische Durchbrüche (8) in der Augenhornhaut (5) mittels in die Augenhornhaut (5) fokussierter Laserstrahlung (3) erzeugt und dabei der Fokuspunkt (7) dreidimensional längs der zu berechnenden Bahnkurve verstellt wird, um die Schnittfläche (9) durch Aneinanderreihung der optischen Durchbrüche (8) zu bilden, wobei der Fokuspunkt auf Basis von Höhenlinien geführt wird, welche in Ebenen liegen, die senkrecht zur ersten Raumrichtung (z) stehen, und wobei bei der Berechnung der Bahnkurve (17, 17.1, 17.2, 17.3) die Verformung der Augenhornhaut (5) berücksichtigt wird, indem eine Koordinatentransformation verwendet wird, die lautet

$$\varphi' = \varphi$$

$$\alpha' \cdot R' = \alpha \cdot R$$

$$R_G - R' = R_{Cv} - R \ ,$$

wobei $\alpha$, $\varphi$, R Kugelkoordinaten vor der Verformung der Augenhornhaut (5), $\alpha'$, $\varphi'$, R' Kugelkoordinaten nach der Verformung der Augenhornhaut (5), $R_{Cv}$ ein Krümmungsradius einer Oberfläche der unverformten Augenhornhaut (5) und $R_G$ ein Krümmungsradius des Kontaktglases (21) sind.

10. Verfahren nach Anspruch 9, **dadurch gekennzeichnet, dass** die Steuerdaten für den Fokuspunkt (7) den übrigen zwei Raumrichtungen auf konzentrischen Höhenlinien der Schnittfläche verläuft.

11. Verfahren nach Anspruch 9, **dadurch gekennzeichnet, dass** die Steuerdaten die einzelnen Höhenlinien nicht als geschlossene Kurven vorgeben und benachbarte Höhenlinien durch einen gleitenden Übergang (18.1, 18.2, 18.3) verbindet.

12. Verfahren nach Anspruch 10 oder 11, **dadurch gekennzeichnet, dass** die Abstände der Höhenlinien (17) in der ersten Raumrichtung so gewählt werden, dass die mittleren Abstände zwischen benachbarten der Höhenlinien (17) im Wesentlichen, insbesondere innerhalb $\pm$ 10%, konstant sind.

13. Verfahren nach Anspruch 9, **dadurch gekennzeichnet, dass** die Bahnkurve einer Ellipsoid-Spirale auf Basis der Höhenlinien folgt.

14. Verfahren nach einem der Ansprüche 9 bis 13, dadurch gegenzeichnet, dass bei höheren Krümmungsordnungen der Schnittfläche (9) die Höhenlinien (17) durch Schneiden einer um höhere Krümmungsordnungen bereinigten gekrümmten Schnittflächen (9) mit Ebenen senkrecht zur ersten Raumrichtung (z) bestimmt werden.

15. Verfahren nach Anspruch 14, dadurch gegenzeichnet, dass die Steuerdaten die Verstellung in der ersten Raumrichtung (z) gemäß dem Einfluss der höheren Krümmungsordnungen variieren, während sie den Fokuspunkt (7) in den übrigen zwei Raumrichtungen (x, y) gemäß der Höhenlinien (17) verstellen, die der bereinigten Schnittfläche (9) ohne höhere Krümmungsordnungen zugeordnet sind.

**Claims**

1. Device for forming a curved excision (9) in the cornea (5), comprising

   - a laser beam source (S), which focuses laser radiation (3) into the cornea (5) and produces optical breakthroughs (8) there, wherein a curved contact lens (21) for pressing onto a surface of the cornea (5) deforms the latter into a specific curved shape,
   - a scanning device (6, 10), which three-dimensionally adjusts the focal point (7), and
   - a control device (2), which activates the scanning device (6, 10) in order to form the excision (9) by arranging the optical breakthroughs (8) in series in the cornea (5), are provided,
   - wherein the scanning device (6, 10) has an adjustable optical unit (6) for adjusting the focal point (7) in a first spatial direction (z), and wherein the control device (2) activates the scanning device (6, 10) in such a way that the focal point is guided on the basis of contour lines which lie in planes that are perpendicular to the first spatial direction (z),
   - wherein the control device (2) activates the scanning device (6, 10) in such a way that the control device (2) takes into account the deformation of the cornea (5) into the curved shape brought about by the contact lens (21) by a coordinate transformation as follows

$$\varphi' \ = \ \varphi$$

$$\alpha' \ \cdot \ R' \ = \ \alpha \ \cdot \ R$$

$$R_G \ - \ R' \ = \ R_{Cv} \ - \ R$$

where $\alpha$, $\varphi$ and R are spherical coordinates before the deformation of the cornea (5), $\alpha'$, $\varphi'$ and R' are spherical coordinates after the deformation of the cornea (5), $R_{Cv}$ is a radius of curvature of the surface of the undeformed cornea (5) and $R_G$ is a radius of curvature of the contact lens (21).

2. Device according to Claim 1, **characterized in that** the control device (2) activates the scanning device (6, 10) in such a way that the focal point is guided in the other two spatial directions on concentric contour lines of the excision.

3. Device according to Claim 1, **characterized in that** the control device (2) activates the scanning device (6, 10) in such a way that the individual contour lines are not created as continuous curves and neighbouring contour lines are connected by a smooth transition (18.1, 18.2, 18.3).

4. Device according to Claim 2 or 3, **characterized in that** the control device (2) chooses the spacings of the contour lines (17) in the first spatial direction in such a way that the average spacings between neighbouring lines of the contour lines (17) are substantially constant, in particular within $\pm$ 10%.

5. Device according to Claim 1, **characterized in that** the control device (2) activates the scanning device (6, 10) in such a way that the focal point (7) follows an ellipsoid spiral on the basis of the contour lines.

6. Device according to Claims 3 to 5, **characterized in that**, in the case of higher orders of curvature of the excision (9), the control device (2) determines the contour lines (17) by cutting a curved excisions (9) corrected by higher orders of curvature, with planes perpendicular to the first spatial direction (z).

7. Device according to Claim 6, **characterized in that** the control device (2) changes the adjustment in the first spatial direction (z) according to the influence of the higher orders of curvature, while it adjusts the focal point (7) in the other two spatial directions (x, y) according to the contour lines (17) that are assigned to the corrected excision (9) without higher orders of curvature.

8. Device according to one of the above claims, **characterized in that** the scanning device (6, 10) has two tilting mirrors (11, 12) with crossed axes of rotation, in order to bring about the adjustment in the other two spatial directions (x, y).

9. Method for generating control data for an ophthalmic-surgical laser device (2) before a surgical intervention,
   wherein the control data prescribe target coordinates which, by a coordinate transformation, take into account a deformation of the cornea (5) by a curved contact lens (21), and
   wherein a trajectory (17, 17.1 17.2, 17.3) that serves for forming a curved excision (9) in the cornea (5) is calculated, wherein, for forming the excision (9), optical breakthroughs (8) are produced in the cornea (5) by means of laser radiation (3) focused into the cornea (5) and the focal point (7) is thereby adjusted three-dimensionally along the trajectory to be calculated, in order to form the excision (9) by arranging the optical breakthroughs (8) in series, wherein the focal point is guided on the basis of contour lines which lie in planes that are perpendicular to the first spatial direction (z), and wherein the deformation of the cornea (5) is taken into account in the calculation of the trajectory (17, 17.1, 17.2, 17.3) by using a coordinate transformation as follows

$$\varphi' = \varphi$$

$$\alpha' \cdot R' = \alpha \cdot R$$

$$R_G - R' = R_{Cv} - R$$

where $\alpha$, $\varphi$ and R are spherical coordinates before the deformation of the cornea (5), $\alpha'$, $\varphi'$ and R' spherical coordinates after the deformation of the cornea (5), $R_{Cv}$ is a radius of curvature of a surface of the undeformed cornea (5) and $R_G$ is a radius of curvature of the contact lens (21).

10. Device according to Claim 9, **characterized in that** the control data for the focal point (7) the other two spatial directions runs on concentric contour lines of the excision.

**11.** Device according to Claim 9, **characterized in that** the control data do not prescribe the individual contour lines as continuous curves and connects neighbouring contour lines by a smooth transition (18.1, 18.2, 18.3).

**12.** Method according to Claim 10 or 11, **characterized in that** the spacings of the contour lines (17) in the first spatial direction are chosen in such a way that the average spacings between neighbouring lines of the contour lines (17) are substantially constant, in particular within $\pm$ 10%.

**13.** Method according to Claim 9, **characterized in that** the trajectory follows an ellipsoid spiral on the basis of the contour lines.

**14.** Method according to one of Claims 9 to 13, **characterized in that**, in the case of higher orders of curvature of the excision (9), the contour lines (17) are determined by cutting a curved excisions (9) corrected by higher orders of curvature, with planes perpendicular to the first spatial direction (z).

**15.** Method according to Claim 14, **characterized in that** the control data vary the adjustment in the first spatial direction (z) according to the influence of the higher orders of curvature, while they adjust the focal point (7) in the other two spatial directions (x, y) according to the contour lines (17) that are assigned to the corrected excision (9) without higher orders of curvature.

**Revendications**

**1.** Dispositif destiné à créer une surface de coupe (9) curviligne dans la cornée de l'oeil (5), comprenant

- une source de rayonnement laser (S), qui focalise un faisceau laser (3) dans la cornée de l'oeil (5) et y provoque des percées optiques (8), un verre de contact (21) curviligne destiné à être pressé sur une surface de la cornée de l'oeil (5) déformant celle-ci pour lui conférer une certaine forme curviligne,
- un système de scanner (6, 10), qui ajuste le point de focalisation (7) en trois dimensions et
- un système de commande (2) qui amorce le système de scanner (6, 10) pour créer la surface de coupe (9) par juxtaposition des percées optiques (8) dans la cornée de l'oeil (5),
- pour ajuster le point de focalisation (7), le système de scanner (6, 10) comportant, dans une première direction spatiale (z), une optique (6) ajustable et le système de commande (2) amorçant le système de scanner (6, 10) de telle sorte que le point de focalisation soit guidé sur la base de courbes de niveau, lesquelles se situent dans des plans qui sont perpendiculaires à la première direction spatiale (z),
- le système de commande (2) amorçant le système de scanner (6, 10) de telle sorte que le système de commande (2) prenne en considération la déformation de la cornée de l'oeil (5) dans la forme curviligne, provoquée par le verre de contact (21) par une transformation de coordonnées, exprimée comme suit :

$$\varphi' = \varphi$$

$$\alpha' \cdot R' = \alpha \cdot R$$

$$R_G - R' = R_{Cv} - R$$

$\alpha$, $\varphi$, R étant des coordonnées sphériques avant la déformation de la cornée de l'oeil (5), $\alpha'$, $\varphi'$, R' étant des coordonnées sphériques après la déformation de la cornée de l'oeil (5), $R_{Cv}$ étant un rayon de courbure de la surface de la cornée de l'oeil (5) non déformée et $R_G$ étant un rayon de courbure du verre de contact (21).

**2.** Dispositif selon la revendication 1, **caractérisé en ce que** le système de commande (2) amorce le système de scanner (6, 10) de telle sorte que le point de focalisation soit guidé dans les deux directions spatiales restantes sur des courbes de niveau concentriques de la surface de coupe.

**3.** Dispositif selon la revendication 1, **caractérisé en ce que** le système de commande (2) amorce le système de scanner (6, 10) de telle sorte que les courbes de niveau individuelles ne soient pas réalisées sous la forme de courbes fermées et que des courbes de niveau voisines soient reliées par un passage progressif (18.1, 18.2, 18.3).

4. Dispositif selon la revendication 2 ou 3, **caractérisé en ce que** le système de commande (2) sélectionne les écarts entre les courbes de niveau (17) dans la première direction spatiale de telle sorte que les écarts moyens entre des courbes de niveau (17) voisines soient sensiblement constants, notamment dans un ordre de ± 10 %.

5. Dispositif selon la revendication 1, **caractérisé en ce que** le système de commande (2) amorce le système de scanner (6, 10) de telle sorte que le point de focalisation (7) suive une spirale ellipsoïdale sur la base des courbes de niveau.

6. Dispositif selon les revendications 3 à 5, **caractérisé en ce que** pour des ordres de courbures assez élevés de la surface de coupe (9), le système de commande (2) détermine les courbes de niveau (17) en recoupant une surfaces de coupe (9) épurée des ordres de courbure plus élevés avec des plans à la perpendiculaire de la direction spatiale (z).

7. Dispositif selon la revendication 6, **caractérisé en ce que** le système de commande (2) modifie l'ajustage dans la première direction spatiale (z) selon l'influence des ordres de courbures plus élevés, alors qu'il ajuste le point de focalisation (7) dans les deux directions spatiales (x, y) restantes, conformément aux courbes de niveau (17) qui sont associées à la surface de coupe (9) épurée, exempte des ordres de courbures plus élevés.

8. Dispositif selon l'une quelconque des revendications précédentes, **caractérisé en ce que** le système de scanner (6, 10) comporte deux miroirs inclinables (11, 12) à axes de rotation croisés, pour provoquer l'ajustage dans les deux directions spatiales (x, y) restantes.

9. Procédé, destiné à générer des données de commande pour un système de laser (2) chirurgical ophtalmique en amont d'une intervention chirurgicale, les données de commande prédéfinissant des coordonnées cibles, qui par une transformation de coordonnées prennent en considération une déformation de la cornée de l'oeil (5) par un verre de contact (21) curviligne et une courbe de trajectoire (17, 17.1, 17.2, 17.3) étant calculée, laquelle sert à créer une surface de coupe (9) curviligne dans la cornée de l'oeil (5), pour créer la surface de coupe (9), des percées optiques (8) étant générées dans la cornée de l'oeil (5) à l'aide d'un faisceau laser (3) focalisé dans la cornée de l'oeil (5) et à cet effet, le point de focalisation (7) étant ajusté en trois dimensions le long de la courbe de trajectoire qui doit être calculée, pour créer la surface de coupe (9) par juxtaposition des percées optiques (8), le point de focalisation étant guidé sur la base de courbes de niveau, lesquelles se situent dans des plans qui sont perpendiculaires à la première direction spatiale (z) et lors du calcul de la courbe de trajectoire (17, 17.1, 17.2, 17.3), la déformation de la cornée de l'oeil (5) étant prise en considération en ce qu'il est utilisé une transformation de coordonnées, exprimée comme suit :

$$\varphi' = \varphi$$

$$\alpha' \cdot R' = \alpha \cdot R$$

$$R_G - R' = R_{Cv} - R$$

$\alpha$, $\varphi$, $R$ étant des coordonnées sphériques avant la déformation de la cornée de l'oeil (5), $\alpha'$, $\varphi'$, $R'$ étant des coordonnées sphériques après la déformation de la cornée de l'oeil (5), $R_{Cv}$ étant un rayon de courbure de la surface de la cornée de l'oeil (5) non déformée et $R_G$ étant un rayon de courbure du verre de contact (21).

10. Procédé selon la revendication 9, **caractérisé en ce que** les données de commande pour le point de focalisation (7) les deux directions spatiales restantes s'écoule sur des courbes de niveau concentriques de la surface de coupe.

11. Procédé selon la revendication 9, **caractérisé en ce que** les données de commande ne prédéfinissent par les courbes de niveau sous la forme de courbes fermées et relie des courbes de niveau voisines par un passage progressif (18.1, 18.2, 18.3).

12. Procédé selon la revendication 10 ou 11, **caractérisé en ce que** les écarts entre les courbes de niveau (17) dans la première direction spatiale sont sélectionnés de telle sorte que les écarts moyens entre des courbes de niveau (17) voisines soient sensiblement constants, notamment dans un ordre de $\pm$ 10 %.

13. Procédé selon la revendication 9, **caractérisé en ce que** la courbe de trajectoire suit une spirale ellipsoïdale sur la base des courbes de niveau.

14. Procédé selon l'une quelconque des revendications 9 à 13, **caractérisé en ce que** pour des ordres de courbure plus élevés de la surface de coupe (9), les courbes de niveau (17) sont déterminées par recoupement d'une surfaces de coupe (9) épurée des ordres de courbure plus élevés avec des plans à la perpendiculaire de la première direction spatiale (z).

15. Procédé selon la revendication 14, **caractérisé en ce que** les données de commande font varier l'ajustage dans la première direction spatiale (z) selon l'influence des ordres de courbure plus élevés, alors qu'elles ajustent le point de focalisation (7) dans les deux autres directions spatiales (x, y) selon les courbes de niveau (17) qui sont associées à la surface de coupe (9) épurée exempte des ordres de courbure plus élevés.

Fig. 1

FIG.2

FIG.3

FIG.4

FIG.8a

FIG.8b

FIG.5

FIG.6

FIG.7

FIG.9

FIG.10

FIG.11

FIG.12

FIG.13

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5984916 A
- US 5984916 B1
- US 6110166 B1
- US 6325792 B1
- US 6110166 A